# FASCICULE DE BREVET EUROPEEN

(11) **EP 3 898 601 B1**
(45) Date de publication et mention de la délivrance du brevet: **26.10.2022**
(21) Numéro de dépôt: 19845765.7
(22) Date de dépôt: 13.12.2019
(51) Int. Cl.: C07D 285/00, C07D 327/00, C08K 5/00, C08K 5/46

(54) **COMPOSÉ POLYSULFURÉ COMME AGENT DE RETICULATION**
POLYSULFIDVERBINDUNG ALS RETIKULIERUNGSMITTEL
POLYSULFIDE COMPOUND AS RETICULATION AGENT

(30) Priorité: 21.12.2018 FR 1873763; 30.01.2019 FR 1900833
(43) Date de publication de la demande: 27.10.2021
(73) Titulaire: COMPAGNIE GENERALE DES ETABLISSEMENTS MICHELIN, 63000 Clermont-Ferrand (FR)
(72) Inventeur: JASSELIN, Adeline, 63040 Clermont-Ferrand CEDEX 9 (FR); NASYBULLIN, Ruslan, 63040 Clermont-Ferrand CEDEX 9 (FR)
(74) Mandataire: Le Cam, Véronique Marie Christine
(86) Numéro de dépôt international: PCT/FR2019/053067
(87) Numéro de publication internationale: WO 2020/128259

(56) Documents cités:
- JP-A- S56 163 131
- US-A1- 2002 107 338
- WONMUN C.: "Synthesis of cyclic polysulfides and their properties as curing agents", RUBBER CHEMISTRY AND TECHNOLOGY, vol. 77, no. 2, 1 janvier 2004 (2004-01-01), pages 380-390, XP002794993,
- HULST R. ET AL.: "Vulcanization of butadiene rubber by means of cyclic disulfides. 2. A 2D solid state HRMAS NMR study on cross-link structures in BR vulcanizates", MACROMOLECULES, vol. 32, 14 octobre 1999 (1999-10-14), pages 7509-7520, XP002794994,

## Description

La présente invention se rapporte aux composés polysulfurés, utilisables notamment pour la réticulation des polymères insaturés, en particulier des élastomères, et plus particulièrement des élastomères diéniques en particulier dans des compositions de caoutchouc destinées par exemple à la fabrication de pneumatiques.

Les polymères insaturés entrent dans la constitution de nombreux objets de la vie courante, tels que des bandages pneumatiques, des semelles de chaussures, des joints d'étanchéité, des colles, des articles en mousse, des profilés pour l'automobile, etc.... Ce sont des macromolécules comportant des doubles liaisons carbone-carbone, également appelées insaturations, dans leur chaîne principale et/ou dans leur(s) chaîne(s) latérale(s).

Il est connu d'accroitre ou de modifier les propriétés mécaniques de ces polymères insaturés en créant des ponts entre leurs chaînes (réticulation). Il existe un certain nombre de procédés permettant la création de ces ponts, le plus connu étant la vulcanisation.

Le principe de la vulcanisation, aussi appelé réticulation par le soufre, réside dans la création de ponts de soufre entre différentes chaînes de macromolécules par réaction sur les doubles liaisons de ces chaînes d'un agent de vulcanisation. L'agent de vulcanisation, aussi appelé donneur de soufre, est un composé qui est capable de libérer du soufre par chauffage à la température de vulcanisation. L'agent de vulcanisation le plus connu et le plus couramment utilisé est le soufre élémentaire.

Il est cependant connu que la vulcanisation au soufre élémentaire a pour inconvénient de conduire à une résistance limitée des vulcanisats obtenus, dû au vieillissement thermique de ces derniers (« thermal ageing »). Lorsqu'ils sont utilisés, les vulcanisats subissent de nombreuses sollicitations, dont des sollicitations thermiques. Ces sollicitations thermiques font l'effet d'une surcuisson, qui lorsque la température du vulcanisât atteint une valeur voisine de la température de vulcanisation, occasionne un phénomène de réversion. La réversion est une rupture du réseau de réticulation par destruction ou raccourcissement des ponts de soufre crées lors de la vulcanisation ; c'est-à-dire une diminution des ponts polysulfures au profit de ponts monosulfures ou disulfures. Cette rupture du réseau modifie défavorablement les propriétés mécaniques du vulcanisat. Ainsi, l'utilisation de soufre élémentaire entraine des contraintes sur le procédé de vulcanisation de compositions comprenant des polymères insaturés, notamment des élastomères en particulier des élastomères diéniques, par rapport à la température de vulcanisation, le temps de cuisson et le module en allongement.

Il reste donc toujours un besoin de disposer d'une composition de polymères insaturés, notamment d'élastomères en particulier d'élastomères diéniques qui ne présente pas un phénomène de réversion ou limité aux hautes températures de réticulation tout en conservant, voire en améliorant les propriétés d'allongement rupture.

Plusieurs solutions ont été mises au point afin de répondre aux inconvénients d'un système de réticulation au soufre élémentaire. On peut citer, par exemple, l'utilisation d'additifs spécifiques appelés agents anti-réversion qui permettent de stabiliser thermiquement les vulcanisats.

Une autre solution a consisté à développer des nouveaux agents de réticulation permettant la formation de ponts de soufre entre les chaînes de macromolécules tout en limitant le phénomène de réversion. A titre d'exemple, le document US2002/107338 décrit la synthèse d'un agent de réticulation : le 1,2,3,4-tétrathiécane.

Il est toujours intéressant pour les industriels de disposer d'agents de réticulation permettant la réticulation de polymères insaturés à haute température, afin de gagner du temps, et d'améliorer encore les propriétés d'allongement rupture des compositions incluant de tels polymères et agents de réticulation.

La Demanderesse a maintenant découvert que les objectifs précités peuvent être atteints, grâce à une nouvelle molécule susceptible d'être utilisée comme agent de réticulation.

Avantageusement cette molécule peut également être utilisée comme agent anti-réversion dans un système de réticulation d'un polymère insaturé.

Ainsi selon un premier aspect, l'invention concerne l'utilisation comme agent de réticulation de polymères insaturés, d'un composé polysulfuré, de formule (I): 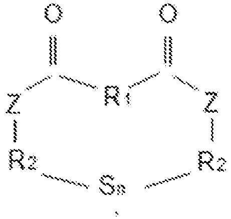 dans laquelle :
n est un nombre supérieur ou égal à 2,
Z représente un atome O ou le groupe -N(H)-,
et R₁ et R₂ représentent chacun un groupement hydrocarboné divalent linéaire ou ramifié, comportant de 1 à 18 atomes de carbone.

Préférentiellement, R₁ et R₂ représentent un radical alkylène comportant de 1 à 18 atomes de carbone, plus préférentiellement de 1 à 10 atomes de carbone et encore plus préférentiellement R₁ et R₂ sont des alkylènes en C1-C7.

Selon une variante préférée de l'invention, le nombre n va de 2 à 6, plus préférentiellement de 2 à 4.

Selon un mode de réalisation préféré de l'invention, Z représente un atome O.

Selon un autre mode de réalisation préféré de l'invention, Z représente un groupe -N(H)-.

Avantageusement le composé polysulfuré consiste en 1,8-dioxa-4,5-dithiacyclotridecane-9,13-dione. L'invention concerne aussi spécifiquement ce composé nouveau.

Selon une autre variante avantageuse de l'invention, le composé polysulfuré consiste en 1,2-dithia-5,11-diazacyclotridecane-6,10-dione. L'invention concerne aussi spécifiquement ce composé nouveau.

Dans la présente description, sauf indication expresse différente, tous les pourcentages (%) indiqués sont des pourcentages en masse.

D'autre part, tout intervalle de valeurs désigné par l'expression « entre a et b » représente le domaine de valeurs allant de plus de « a » à moins de « b » (c'est-à-dire bornes a et b exclues) tandis que tout intervalle de valeurs désigné par l'expression « de a à b » signifie le domaine de valeurs allant de « a » jusqu'à « b » (c'est-à-dire incluant les bornes strictes a et b).

Dans la présente demande, on entend par « pce » (usuellement « phr » en anglais) la partie en poids d'un constituant pour cent parties en poids du ou des élastomères, c'est-à-dire du poids total du ou des élastomères. Lorsque les objets de la présente invention comprennent des compositions dépourvues d'élastomère, l'homme du métier comprendra que le terme « pce » s'entend comme la partie en poids d'un constituant de la composition pour cent parties en poids du ou des polymères insaturés de cette composition.

Dans le cadre de l'invention, les produits contenant du carbone mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. Sont concernés notamment les polymères, les plastifiants, les charges, etc.

Composé polysulfuré de l'invention.

Le premier objet de l'invention est l'utilisation comme agent de réticulation de polymères insaturés, d'un composé polysulfuré de formule (I) 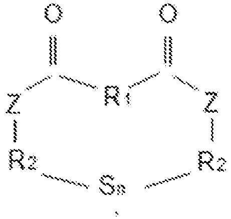 dans laquelle :
n est un nombre supérieur ou égal à 2,
Z représente un atome O ou le groupe -N(H)-,
et R₁ et R₂ représentent chacun un groupement hydrocarboné divalent linéaire ou ramifié, comportant de 1 à 18 atomes de carbone.

Par « groupement hydrocarboné divalent », on entend au sens de la présente invention un groupement comprenant des atomes de carbone et d'hydrogène. Le groupement hydrocarboné peut être éventuellement ramifié, notamment substitué par des groupements alkyles en C1-C6, par le phényle et/ou par le benzyle. Le groupement hydrocarboné peut également et préférentiellement être linéaire, c'est-à-dire qu'il n'est pas ramifié. Il peut être aussi cyclique. Il peut éventuellement comprendre une ou plusieurs doubles liaisons carbone-carbone. Préférentiellement dans la formule générale (I), les radicaux R₁ et R₂ représentent respectivement un radical alkylène comportant de 1 à 18 atomes de carbone, de préférence de 1 à 10 atomes de carbone, plus préférentiellement encore de 1 à 7 atomes de carbone.

De préférence, n représente un nombre compris dans un domaine allant de 2 à 6, plus préférentiellement de 2 à 4. Les composés polysulfurés de formule générale (I), y compris leurs variantes préférées, peuvent être des mélanges de composés de formule générale (I) auquel cas les indices n sont des valeurs moyennes. Ces indices peuvent donc être des nombres entiers comme des nombres fractionnaires. Egalement, le composé polysulfuré peut se présenter sous forme d'un mélange de forme cyclique et non cyclique.

Selon un mode de réalisation préféré de l'invention, le composé polysulfuré consiste en un 1,8-dioxa-4,5-dithiacyclotridecane-9,13-dione.

Selon un mode de réalisation préféré de l'invention, le composé polysulfuré consiste en 1,2-dithia-5,11-diazacyclotridecane-6,10-dione.

### Procédé de synthèse

Les composés polysulfurés utiles pour l'invention peuvent être préparés par tout moyen connu de l'homme de l'art. Par exemple, on peut utiliser un procédé selon le schéma réactionnel suivant.

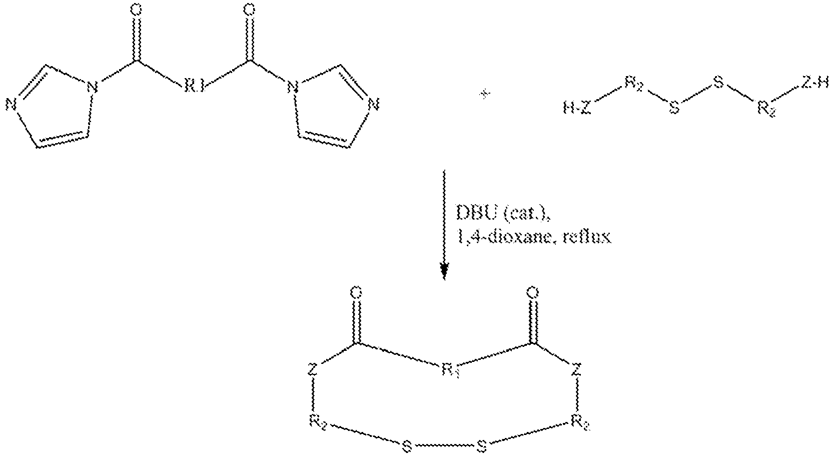

La définition du groupement R₁ et R₂ de la formule générale (I) s'applique également aux groupements représentés ci-dessus.

### Utilisation à titre d'agent de réticulation

Comme indiqué précédemment, le composé polysulfuré trouve une application industrielle avantageuse comme agent de réticulation des polymères insaturés.

Sans que ceci soit limitatif, il peut être notamment utilisé pour la réticulation des élastomères diéniques, par exemple dans des compositions de caoutchouc destinées à la fabrication de pneumatiques.

Par élastomère (ou « caoutchouc », les deux termes étant considérés comme synonymes) du type « diénique », on rappelle ici que doit être compris de manière connue un (on entend un ou plusieurs) élastomère issu au moins en partie (i.e., un homopolymère ou un copolymère) de monomères diènes (monomères porteurs de deux doubles liaisons carbone-carbone, conjuguées ou non).

Pour une telle utilisation, l'élastomère diénique est alors de préférence choisi dans le groupe des élastomères diéniques fortement insaturés constitué par les polybutadiènes (en abrégé BR), les polyisoprènes (IR) de synthèse, le caoutchouc naturel (NR), les copolymères de butadiène, les copolymères d'isoprène et les mélanges de ces élastomères diéniques. De tels copolymères sont plus préférentiellement choisis dans le groupe constitué par les copolymères de butadiène-styrène (SBR), les copolymères d'isoprène-butadiène (BIR), les copolymères d'isoprène-styrène (SIR), les copolymères d'isoprène-butadiène-styrène (SBIR), les copolymères de butadiène-acrylonitrile (NBR), les copolymères de butadiène-styrène-acrylonitrile (NSBR) ou un mélange de deux ou plus de ces composés. Dans le cas d'un élastomère SBR (ESBR ou SSBR), on utilise notamment un SBR ayant une teneur en styrène moyenne, par exemple comprise entre 20% et 35% en poids, ou une teneur en styrène élevée, par exemple de 35 à 45%, une teneur en liaisons vinyliques de la partie butadiénique comprise entre 15% et 70%, une teneur (% molaire) en liaisons trans 1,4 comprise entre 15% et 75% et une Tg comprise entre -10°C et -55°C ; un tel SBR peut être avantageusement utilisé en mélange avec un BR possédant de préférence plus de 90% (% molaire) de liaisons cis 1,4. Plus préférentiellement encore, l'élastomère diénique est le caoutchouc naturel ou un polyisoprène de synthèse.

Les compositions de caoutchouc pour cette utilisation, comprennent au moins une charge renforçante telle qu'une charge organique, par exemple le noir de carbone ou une charge inorganique renforçante, telle que la silice. En particulier lorsqu'une charge inorganique renforçante est présente dans la composition, en particulier une silice, on peut utiliser de manière connue un agent de couplage (ou agent de liaison) au moins bifonctionnel destiné à assurer une connexion suffisante, de nature chimique et/ou physique, entre la charge inorganique (surface de ses particules) et l'élastomère diénique, en particulier des organosilanes ou des polyorganosiloxanes bifonctionnels.

Le composé polysulfuré de formule générale (I) est un agent de réticulation. Il peut avantageusement remplacer le soufre élémentaire utilisé usuellement en réticulation. Il peut également être utilisé en complément d'un système de réticulation, comme agent anti-réversion.

Préférentiellement, la composition présente un taux de composé polysulfuré de formule générale (I) compris dans un domaine allant de 0,5 à 10 pce, de préférence allant de 0,5 à 5 pce, et plus préférentiellement de 0,5 à 3 pce.

Ces co-agents, optionnels, peuvent être des activateurs de vulcanisation, des accélérateurs de vulcanisation, bien connus de l'homme du métier.

A titre d'activateurs de vulcanisation, on peut citer l'oxyde de zinc et les dérivés d'acide stéarique. On entend par « dérivé d'acide stéarique », de l'acide stéarique ou un sel d'acide stéarique, tous deux étant bien connus de l'homme du métier. A titre d'exemple de sel d'acide stéarique utilisable dans le cadre de la présente invention, on peut citer notamment le stéarate de zinc ou de magnésium.

A titre d'accélérateurs de vulcanisation bien connus de l'homme du métier, on choisira de préférence dans le groupe constitué par les thiazoles, les sulfénamides, les guanidines, les amines, les aldéhyde-amines, les dithiophosphates, les xanthates, les thiurames, les dithiocarbamates et leurs mélanges. En particulier, on préfère pour la présente invention, le benzothiazyl-2-cyclohexyl sulfénamide (CBS), le benzothiazyldicyclohexy-1 sulfénamide (DCBS), le benzothiazoyl-2-tert.-butyl sulfénamide (TBBS), le 2-mercaptobenzothiazole (MBT), le disulfure de benzothiazole (MBTS), le sel de zinc ou de sodium de 2-mercaptobenzothiazole (ZMBT), le morpholide de benzothiazyl-2-sulfène (MBS), la diphényle guanidine (DPG), la triphényle guanidine (TPG), la diorthotolyl guanidine (DOTG), la o-tolylbiguanide (OTBG), le disulfure de benzothiazole (MBTS), le disuflure de tetraméthylthiurame (TMTD), le disulfure de tetrabenzylthiurame (TBzTD), le dibenzyldithiocarbamate de zinc (ZBEC), le zinc N,N'-dimethylcarbamodithioates (ZDMC), le zinc N,N'-diethylcarbamodithioates (ZDEC), le zinc N,N'-dibutylcarbamodithioates (ZDBC), le zinc N,N'-dibenzylcarbamodithioates (ZDBC), le zinc isopropylxanthate (ZIX) et leurs mélanges.

### Exemples

Dans les exemples de réalisation qui suivent, l'invention est mise en œuvre avec deux composés polysulfures : le 1,8-dioxa-4,5-dithiacyclotridecane-9,13-dione et le 1,2-dithia-5,11-diazacyclotridecane-6,10-dione.

### A/ Synthèse du composé polysulfuré 1 conforme à l'invention: 1,8-dioxa-4,5-dithiacyclotridecane-9,13-dione

Le 1,8-dioxa-4,5-dithiacyclotridecane-9,13-dione est synthétisé selon le schéma ci-dessous et selon le protocole de synthèse suivant :

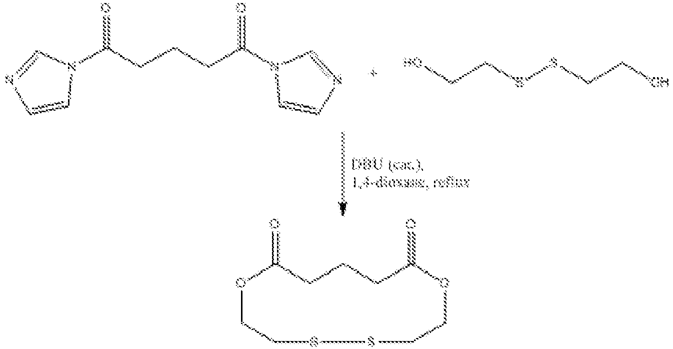

### Synthèse du précurseur : 1,5-di(1H-imidazol-1-yl)pentane-1,5-dione

La synthèse de ce précurseur est décrite dans la littérature notamment dans Synth. Commun. 1985, 15, 1159-1164 ; J. Chem. Soc. Perkin Trans. 2 1996, 2673-2679 ; J. Am. Chem. Soc. 1989, 111, 6428-6429, selon le schema réactionnel suivant :

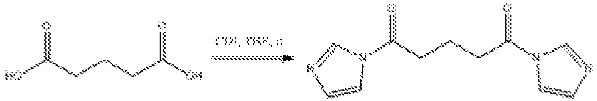

L'acide glutarique (5.00g; 38 mmol) est solubilisée dans le THF anhydre (20 mL). Le 1,1'-carbonyldiimidazole (16.57g; 102 mmol) dans le THF anhydre (80 mL) est ajouté au mélange. Le milieu réactionnel est agité à température ambiante pendant 4h. Le précipité est filtré et lavé par l'éther de pétrole et séché à l'air.

Un solide blanc (8.267 g, 35.60 mmol, rendement 94%) du point de fusion 161-163°C (avec la décomposition) est obtenu.

La pureté molaire est supérieure à 87 % mol (RMN 1H).

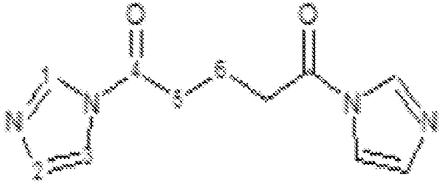

| N° | δ ⁸H (ppm) | δ ¹³C (ppm) |
|---|---|---|
| 1 | 8.34 | 136.8 |
| 2 | 7.01 | 130.2 |
| 3 | 7.64 | 116.4 |
| 4 | / | 170.0 |
| 5 | 3.09 | 33.3 |
| 6 | 1.99 | 17.8 |

| | | |
|---|---|---|
| Solvant: DMSO | | |

### Synthèse du composé polysulfuré 1 : 1,8-dioxa-4,5-dithiacyclotridecane-9,13-dione

A une suspension de 1,5-di(1H-imidazol-1-yl)pentane-1,5-dione (8.264g; 35.6 mmol) dans le 1,4-dioxane anhydre (100 mL) est ajouté le 2-hydroxyethyl disulfide (5.49g; 35.6 mmol) à température ambiante sous l'agitation intense. La catalyses de 1,8-diazabicyclo[5.4.0]undec-7-ene (3 gouttes) est ajouté au milieu réactionnel et le mélange est chauffé (Tbain = 80°C) pendant 15-20 heures. Apres d'achèvement de la réaction le solvant est évaporé pour obtenir d'huile jaune (14 g) qui se cristallise rapidement à température ambiante. Le produit cru est traité pendant 2 heures à température ambiante par l'EtOH (50 mL) et filtré. Cette procédure est répétée 3 fois. Le précipité est séché sous vide à température ambiante (à 7×10-2 mbar).

Un solide blanc (7.611 g, 30.40 mmol, rendement 85%) du point de fusion 47-49°C est obtenu.

La pureté molaire est supérieure à 93 % mol (RMN 1H)

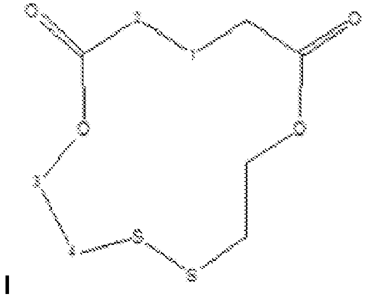

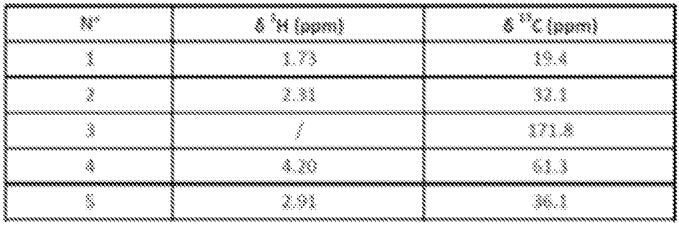

Solvant: DMSO

B/ Synthèse du composé polysulfuré 2 conforme à l'invention : 1,2-dithia-5,11-diazacyclotridecane-6,10-dione Le 1,2-dithia-5,11-diazacyclotridecane-6,10-dione est synthétisé selon le schéma réactionnel ci-dessous et selon le protocole de synthèse suivant :

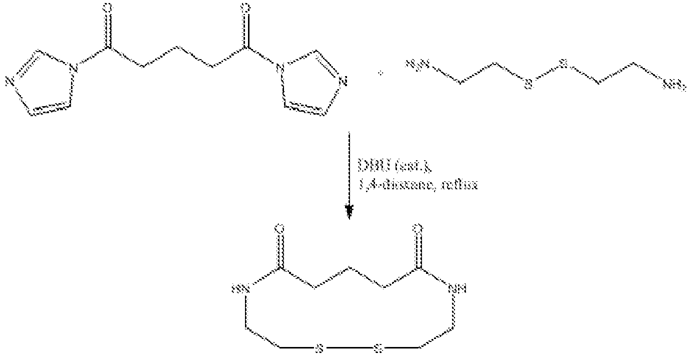

### La synthèse du précurseur : 1,5-di(1H-imidazol-1-yl)pentane-1,5-dione est réalisée comme décrit dans la partie A/

La synthèse du précurseur cystamine est réalisée à partir de cystamine dihydrochloride commerciale , tel que décrit dans la littérature, on citera notamment .Chin. J. Chem. 2011, 29, 531-538 ; J. Organomet. Chem. 2005, 690, 2543-2547, selon le schéma réactionnel suivant :

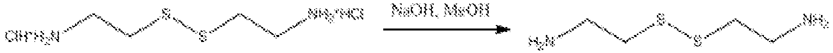

### Synthèse du composé polysulfuré 2 : 1,2-dithia-5,11-diazacyclotridecane-6,10-dione

A une suspension de 1,5-di(1H-imidazol-1-yl)pentane-1,5-dione (8.05g; 34.7 mmol) dans le 1,4-dioxane anhydre (80 mL) est ajouté le cystamine (5.28g; 34.7 mmol) à température ambiante sous l'agitation intense. La catalyses de 1,8-diazabicyclo[5.4.0]undec-7-ene (3 gouttes) est ajouté au milieu réactionnel et le mélange est chauffé à température d'ébullition pendant 18 heures. Après l'achèvement de la réaction le mélange réactionnel est dilué par l'eau (500 mL) et mélangé pendant 3 heures à température ambiante.

Le précipité amorphe est filtré et lavé par l'eau. Le produit est séché sous l'air à température ambiante.

Un solide jaune (7.778 g, rendement 90%) du point de fusion 196-197°C est obtenu.

La pureté molaire est supérieure à 95 % mol (RMN 1H).
Tableau d'attribution :

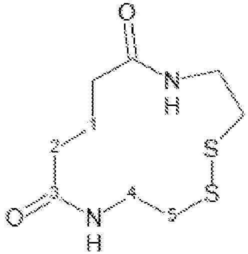

| | δ¹H (ppm) | δ¹³C(ppm) |
|---|---|---|
| 1 | 1.68 | 20.8 |
| 2 | 2.03 | 34.3 |
| 3 | / | 171.4 |
| 4 | 3.28 | 37.6 |
| 5 | 2.74 | 37.2 |

| | | |
|---|---|---|
| Solvent: DMSO | | |

### C/ Synthèse du composé polysulfuré 3 non conforme à l'invention: 1 ,2,3,4-tetrathiecane

Le 1,2,3,4-tétrathiécane est synthétisé selon le schéma réactionnel ci-dessous et selon le protocole de synthèse suivant :

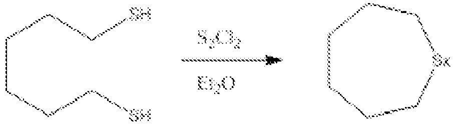

A une solution de 1,6-hexanedithiol (8,0 g, 0,053 mol) dans l'éther éthylique (150 ml) à -3°C (température bain), est ajoutée pendant 60 minutes, goutte à goutte, une solution de S2Cl2 (7,19 g, 0.053 mmol) dans l'éther éthylique (80 ml). La température du milieu réactionnel est ramenée à température ambiante (à 23°C) sur 1,5 heures. Puis, le milieu est chauffé 1,5 heures à 30°C (température bain). Le précipité formé est filtré puis solubilisé dans le dichlorométhane (200 ml) à 40°C. Le dichlorométhane est évaporé sous pression réduite (1-2 mbar, 35-40°C).

Après séchage du produit pendant 6 heures sous vide (1-2 mbar, 40°C), une huile très visqueuse est obtenue (8,61 g, rendement de 77%).

La pureté molaire est supérieure à 98 % mol. (RMN 1H).

La distribution des x est suivante : 0,3% pour x=2; 1,2 % pour x=3; 98,5% pour x >4

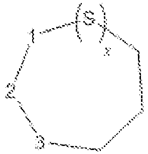

| N° | **δ¹H (ppm)** | **δ** ¹³**C (pmm)** |
|---|---|---|
| 1 | x=2 : 2,63 ; x=3 : 2,82 ; x=4+ : 2,90 | x=2 : 38,5 ; x=3 : 38,5 ; x=4+ : 39,6 |
| 2 | 1,74 | 28,5 |
| 3 | 1,42 | 27,5 |

### D/ Compositions

Pour les besoins de ces essais, des compositions de caoutchouc ont été préparées comme précisé ci-dessous, dont les formulations sont données dans le tableau 1 ; le taux des différents produits est exprimé en pce (parties en poids pour cent parties en poids d'élastomère).

Pour la fabrication de ces compositions, on a procédé de la manière suivante: on a introduit dans un mélangeur interne, dont la température initiale de cuve était d'environ 50°C, successivement la charge renforçante (noir de carbone), l'élastomère diénique, ainsi que les divers autres ingrédients à l'exception du système de réticulation ; le mélangeur était ainsi rempli à environ 70% (% en volume). On a conduit alors un travail thermomécanique (phase non-productive) en une étape d'environ 3 à 5 min, jusqu'à atteindre une température maximale de "tombée" de 160°C. On a récupéré le mélange ainsi obtenu, on l'a refroidi puis on a incorporé le système de vulcanisation sur un mélangeur externe (homo-finisseur) à 40°C, en mélangeant le tout (phase productive) pendant quelques minutes.

Les compositions sont ensuite calandrées sous forme de plaques de caoutchouc d'épaisseur de 2 à 3 mm.

Les compositions C1 et C2 utilisant des composés polysulfurés conformes à l'invention, ont été comparées à deux compositions témoin, T1 et T2, de formulation similaire préparées de manière identique, dont seul le système de réticulation diffère.

**Tableau 1**

| Compositions | T1 | T2 | C1 | C2 |
|---|---|---|---|---|
| NR (1) | 100 | 100 | 100 | 100 |
| Silice (2) | 45 | 45 | 45 | 45 |
| Silane (3) | 13 | 13 | 13 | 13 |
| Soufre | 1 | - | - | - |
| Composé polysulfuré 1 (4) | - | - | 5,75 | - |
| Composé polysulfuré 2 (5) | - | - | - | 5,71 |
| Composé polysulfuré 3 (6) | - | 4,87 | | - |
| Oxyde de zinc (7) | 6 | 6 | 6 | 6 |
| Acide stéarique (8) | 2 | 2 | 2 | 2 |
| 6PPD (9) | 2 | 2 | 2 | 2 |
| Accélérateur (10) | 0,75 | 0,75 | 0,75 | 0,75 |
| Accélérateur (11) | 2 | 2 | 2 | 2 |
| Accélérateur (12) | 2 | 2 | 2 | 2 |

| | | | | |
|---|---|---|---|---|
| (1) Caoutchouc naturel (2) Silice « Zeosil 1165MP » commercialisée par la société Solvay ; (3) tétrasulfure de bis(3-triéthoxysilylpropyl), TESPT, "SI69" commercialisé par la société Evonik 1,8-dithiacyclotetradecane 1,8-disulfide (4) Composé polysulfuré 1 : 1,8-dioxa-4,5-dithiacyclotridecane-9,13-dione (5) Composé polysulfuré 2 : 1,2-dithia-5,11-diazacyclotridecane-6,10-dione (6) Composé polysulfuré 3 : 1 ,2,3,4-tetrathiecane (7) Oxyde de zinc de grade industriel - société Umicore ; (8) Stéarine « Pristerene 4931 » de la société Uniqema ; (9) N-(1,3-diméthylbutyl)-N'-phényl-p-phénylènediamine (Santoflex 6-PPD) de la société Flexsys ; (10) Diphenylguanidine (11)N-cyclohexyl-2-benzothiazol-sulfénamide (« VULKACIT^{®} » de la société Lanxess); (12) Tétrabenzylthiuram disulfide | | | | |

Les propriétés de ces différentes compositions après cuisson respectivement à des températures de 150°C ou de 190°C ont été évaluées et sont présentées dans le tableau 2 ci-dessous.

### Test de traction

Les propriétés mesurées sont les modules des compositions, tels qu'ils sont déterminés lors d'essais de traction. Ces essais permettent de déterminer les contraintes d'élasticité et les propriétés à la rupture. Ils sont effectués conformément à la norme française NF T 46-002 de septembre 1988. On mesure en seconde élongation (i.e. après un cycle d'accommodation) les allongements à la rupture (en %). Les mesures d'allongement à la rupture (notés AR) sont effectuées dans les conditions normales de température (23±2°C) et d'hygrométrie (50+5% d'humidité relative), selon la norme française NF T 40-101 (décembre 1979), et également à 100°C. Les modules sécants nominaux (ou contraintes apparentes, en MPa, rapportées à la déformation, sans unité) à 10 %, 100% ou à 300% d'allongement (notés MA10, MA100 et MA300) et les contraintes vraies à la rupture (en MPa) peuvent également être mesurées.

**Tableau 2**

| Compositions | T1 | T2 | C1 | C2 |
|---|---|---|---|---|
| Après cuisson à 150°C | | | | |
| Allongement rupture (%, 23°C) | 110 | 170 | 415 | 356 |
| Contrainte rutpure (MPa, 23°C) | 8,8 | 17,4 | 25,5 | 22,3 |
| Allongement rupture (%, 100°C) | 97 | 89 | 356 | 276 |
| Contrainte rutpure (MPa, 100°C) | 7,3 | 6,9 | 13,5 | 10,4 |

| Après cuisson à 190°C | | | | |
|---|---|---|---|---|
| Allongement rupture (%, 23°C) | 180 | 175 | 411 | 305 |
| Contrainte rutpure (MPa, 23°C) | 12,7 | 14,9 | 24,4 | 18,8 |
| Allongement rupture (%, 100°C) | 115 | 118 | 324 | 281 |
| Contrainte rutpure (MPa, 100°C) | 6,0 | 7,7 | 11,8 | 10,5 |

L'examen du tableau 2 montre que de façon très surprenante, les compositions C1 et C2 incluant les composés polysulfurés conformes à l'invention, présentent de propriétés d'allongement à la rupture et de contrainte à la rupture très supérieures à chacune des compositions témoins T1 et T2. On constate de plus que ce phénomène apparaît aussi bien après cuisson à 150°C, qu'après cuisson à 190°C.

Ainsi il apparaît que les compositions qui comportent un composé polysulfuré de formule générale (I) conformes à l'invention, permettent de façon étonnante non seulement de maintenir mais d'améliorer significativement les propriétés à la rupture de telles compositions lors d'une réticulation à haute température.

## Revendications

1. Utilisation d'au moins un composé polysulfuré de formule générale (I) comme agent de réticulation de polymères insaturés : 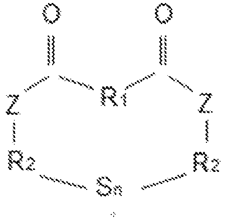 dans laquelle :
n est un nombre supérieur ou égal à 2,
Z représente un atome O ou le groupe -N(H)-,
et R₁ et R₂ représentent chacun un groupement hydrocarboné divalent linéaire ou ramifié, comportant de 1 à 18 atomes de carbone.

2. Utilisation selon la revendication 1, dans laquelle R₁ et R₂ représentent un radical alkylène comportant de 1 à 18 atomes de carbone, de préférence de 1 à 10 atomes de carbone.

3. Utilisation selon la revendication 2, dans laquelle R₁ et R₂ sont des alkylènes en C1-C7.

4. Utilisation selon l'une quelconque des revendications précédentes, dans laquelle n représente un nombre allant de 2 à 6, plus préférentiellement de 2 à 4.

5. Utilisation selon l'une quelconque des revendications précédentes, dans laquelle Z représente un atome O.

6. Utilisation selon l'une quelconque des revendications 1 à 4, dans laquelle Z représente un groupe - N(H)-.

7. Composé 1,8-dioxa-4,5-dithiacyclotridecane-9,13-dione.

8. Composé 1,2-dithia-5,11-diazacyclotridecane-6,10-dione.

9. Utilisation comme agent de réticulation de polymères insaturés, d'au moins un composé selon l'une quelconque des revendications 7 ou 8.

## Patentansprüche

1. Verwendung mindestens einer Polysulfidverbindung der allgemeinen Formel (I) als Vernetzungsmittel für ungesättigte Polymere: 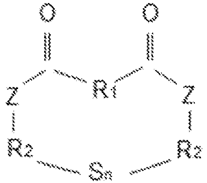 in der:
n eine ganze Zahl größer oder gleich 2 ist,
Z für ein O-Atom oder die Gruppe -N(H)- steht und R₁ und R₂ jeweils für eine lineare oder
verzweigte zweiwertige Kohlenwasserstoffgruppe mit 1 bis 18 Kohlenstoffatomen stehen.

2. Verwendung nach Anspruch 1, wobei R₁ und R₂ für einen Alkylenrest mit 1 bis 18 Kohlenstoffatomen, vorzugsweise 1 bis 10 Kohlenstoffatomen, stehen.

3. Verwendung nach Anspruch 2, wobei R₁ und R₂ für C1-C7-Alkylengruppen stehen.

4. Verwendung nach einem der vorhergehenden Ansprüche, wobei n für eine ganze Zahl im Bereich von 2 bis 6, weiter bevorzugt von 2 bis 4, steht.

5. Verwendung nach einem der vorhergehenden Ansprüche, wobei Z für ein O-Atom steht.

6. Verwendung nach einem der Ansprüche 1 bis 4, wobei Z für eine Gruppe -N(H)- steht.

7. Verbindung 1,8-Dioxa-4,5-dithiacyclotridecan-9,13-dion.

8. Verbindung 1,2-Dithia-5,11-diazacyclotridecan-6,10-dion.

9. Verwendung mindestens einer Verbindung nach einem der Ansprüche 7 oder 8 als Vernetzungsmittel für ungesättigte Polymere.

## Claims

1. Use of at least one polysulfide composition of general formula (I) as a crosslinking agent in unsaturated polymers: 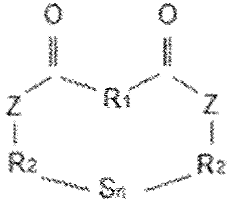 wherein:
n is a number greater than or equal to 2,
Z represents an oxygen atom or the -N(H)- group,
and R₁ and R₂ each represent a divalent, linear or branched hydrocarbyl group containing 1 to 18 carbon atoms.

2. Use according to Claim 1, wherein R₁ and R₂ represent an alkylene radical containing 1 to 18 carbon atoms, preferably 1 to 10 carbon atoms.

3. Use according to Claim 2, wherein R₁ and R₂ are C1-C7 alkylenes.

4. Use according to any one of the preceding claims, wherein n represents a number ranging from 2 to 6, more preferably from 2 to 4.

5. Use according to any one of the preceding claims, wherein Z represents an oxygen atom.

6. Use according to any one of Claims 1 to 4, wherein Z represents an -N(H)- group.

7. Compound 1,8-dioxa-4,5-dithiacyclotridecane-9,13-dione.

8. Compound 1,2-dithia-5,11-diazacyclotridecane-6,10-dione.

9. Use as crosslinking agent in unsaturated polymers of at least one compound according to either one of Claims 7 and 8.
